# EUROPEAN PATENT APPLICATION

(11) **EP 1 243 574 A1**
(43) Date of publication of application: **25.09.2002**
(21) Application number: 00972911.2
(22) Date of filing: 19.10.2000
(51) Int. Cl.: C07C 45/74, C07C 47/232, B01J 29/82, B01J 29/83, B01J 29/85

(54) **METHOD AND (ACID AND BASIC) CATALYSTS FOR OBTAINING ALPHA-ALKYL CINAMALDEHYDES**

(30) Priority: 19.10.1999 ES 9902349
(71) Applicant: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: Corma Canos, Avelino Univ. Poli. de Valencia, Calle los Naranjos 46022 Valencia (ES); Iborra Chornet, Sara Univ. Poli. de Valencia, Calle los Naranjos 46022 Valencia (ES); Climent Olmedo, Maria J. Uni. P. de Valencia, Calle los Naranjos 46022 Valencia (ES); Guil-Lopez, Ruth Univ. Poli. de Valencia, Calle los Naranjos 46022 Valencia (ES)
(74) Representative: Ungria Lopez, Javier
(86) International application number: ES0000404
(87) International publication number: WO01028968

(57) **Abstract**

Heterogeneous acid catalysts are used in a process for the selective obtainment of α-alkylcinnamaldehydes, and more specifically Jasmine aldehyde, by direct reaction between n-alkylaldehydes and benzaldehyde or a benzaldehyde substituted in its aromatic ring: aluminum phosphates (AlPO), silicoaluminophosphates (SAPO), and silicoaluminates (MAS) or basic catalysts corresponding to nitrides of such acid catalysts: oxynitrided aluminum phosphates (AlPON), oxynitrided silicoaluminophosphates (SAPON) and oxynitrided silicoaluminates (SAMON).

## Description

### TECHNICAL FIELD OF THE INVENTION

The present invention fits in the technical field of the preparation of α-alkylcinnamaldehydes by reaction between alkylaldehydes and benzaldehyde or derivatives thereof in order to obtain α-substituted cinnamaldehydes, especially the preparation of α-n-amylcinnaimaldehyde (Jasmine aldehyde) by means of direct aldol condensations and catalyzed by solid catalysts with acid or basic characteristics.

### PRIOR ART OF THE INVENTION

α-n-amylcinnamaldehyde has a violet smell and is a substance commonly used in perfumery. It may be obtained by means of aldol condensation between an alkylaldehyde such as for example a heptanal and benzaldehyde using alkaline catalysts. The problem of this type of condensation is that both reagents may undergo secondary reactions giving rise to by-products that reduce the yield of alkylcinnamaldehyde and that may also provide an unpleasant smell, thus reducing the quality of the perfume.

One of these undesired reactions is the autocondensation of alkylaldehyde, whose formation may be inhibited to a large degree, by keeping the concentration of this reagent very low with respect to that of the benzaldehyde in the reaction medium. This process thus requires the heptanal to be added slowly, which leads to the use of batch reactions with long reaction times and a certain difficulty in the use of a continuous reactor.

Another type of by-product that is formed in these conditions corresponds to the ones derived from the instability of benzaldehyde which tends to undergo Cannizzaro disproportionation giving rise to benzyl alcohol and benzoic acid which in turn reacts with the basic catalyst thus causing the neutralization of the catalyst.

Patent DD-A-11191 describes a process for the obtainment of Jasmine aldehyde wherein carboxylic acid Ni, Co or Fe salts are used as catalysts. The reaction is carried out at a temperature of 180-190°C, in the presence of toluene in order to facilitate azeotropic distillation of water and the heptanal is added slowly to the reaction medium. Hence, yields of Jasmine aldehyde is the range of 80% are obtained.

Patent DD-A-287712 describes a process wherein the aldol condensation between heptanal and benzaldehyde is carried out in the presence of titanium compounds (tetraisopropyl titanium) and toluene as an azeotropic agent. Yields of Jasmine aldehyde in the range of 56% are obtained.

Patent application EP-A-0392579 describes another process for the selective obtainment of α-cinnamaldehyes by aldol condensation, in two steps using glycols (especially ethylene glycol) as solvents and sodium or potassium hydroxide as catalysts. Yields of α-n-cinnamaldehyde in the range of 90% are obtained.

Patent application EP-A-0771780 describes a method for the preparation of α-alkylcinnamaldehydes by aldol condensation between benzaldehyde and an alkanal, using amines as homogeneous catalysts. Aldol condensation is carried out in the absence of a solvent and by slowly adding the alkanal (to which a small amount of carboxylic acid has been added) to the mixture that contains benzaldehyde and the amine. Selectivities between 55 and 96% are obtained depending on the type of amine used as a catalyst.

P. Mastagli et al. in Bull. Soc. Chim. France, 1955, p. 268 describe the preparation of Jasmine aldehyde using anionic exchange resins (IR-4B) and the yields obtained vary between 2 and 12%.

P. Mastagli also describes in Compt. Rend. 1957 vol. 244, p. 207, a process starting with n-alkylaldehyde acetal and benzaldehyde using a mixture of an acid catalyst (cationic exchange resin) and a basic catalyst (anionic exchange resin) capable of producing hydrolysis and aldol condensation, respectively, as a catalyst. The yields of Jasmine aldehyde obtained vary between 5 and 28%.

A. Sakar et al. describe in Ind. J. Chem., 1986, vol. 25, p. 656, aldol condensation using potassium carbonate and a phase transfer catalyst such as benzyltriethylammonium chloride. On the other hand, D. Abenhaim et al. in Synthetic Comm., 1994, vol. 24, p. 1199 describe condensation using the same type of catalysts but under the effect of microwaves. In both cases yields of Jasmine aldehyde in the range of 80% are obtained.

On the other hand, patent application WO-A-9835928 describes the selective obtainment of α-alkylcinnamaldehydes, specifically, Jasmine aldehyde, by direct reaction between n-alkylaldehyde acetal (heptanal) and benzaldehyde using zeolite acids and medium and large pore zeotypes with rings of 10 and preferably 12 members as catalysts, as well as mesoporous molecular sieve. These acid catalysts consecutively cause slow hydrolysis of acetal and mixed aldol condensation, keeping at all times a low concentration of n-alkylaldehyde with respect to benzaldehyde, thus obtaining α-alkylcinnamaldehydes with high yields and selectivities.

### DESCRIPTION OF THE INVENTION

The purpose of the present invention is to overcome the inconveniences of the above-cited prior art, by means of a process for the preparation of an α-substituted cinnamaldehyde by means of a mixed aldol condensation reaction between an alkylaldehyde and benzaldehyde or a benzaldehyde substituted in its aromatic ring, catalyzed by a solid catalyst, in whose process the catalyst is selected among at least a heterogeneous acid catalyst, at least a nitride of a heterogeneous acid and combinations thereof and the condensation reaction is direct.

In accordance with the invention, the heterogeneous acid catalyst is selected among aluminum phosphates (AlPO), silicoaluminophosphates (SAPO) and mesoporous amorphous alumina-silica (MAS) and because the heterogeneous acid catalyst nitride is an oxynitride selected among oxinitrided aluminum phosphates (AlPON), silicoaluminophosphates (SAPON) and oxynitrided mesoporous amorphous alumina-silica (SAMON). The use of these catalysts as selective catalysts in the obtainment of α-alkylcinnamaldehydes and especially Jasmine aldehyde, by mixed aldol condensation between an alkylaldehyde and benzaldehyde or benzaldehyde substituted in its aromatic rings is especially advantageous which is shown by the fact that, in all of the experiments carried out, the conversion was of 100% with yields higher than 80%.

The condensation reaction is carried out according to conventional processes in a stirred tank type continuous or discontinuous reactor, or in a fixed continuous or fluidized bed continuous reactor, wherein the catalyst is. The reaction can be carried out in an inert atmosphere at atmospheric pressure or in a reactor at a pressure between 2 and 10 atm. Preferably, it is carried out at atmospheric pressure, under an inert atmosphere, for example nitrogen and at a temperature between 25 and 300°C, preferably between 75 and 175°C.

The amount of catalyst is between 0.05 and 30% with respect to the total weight of reagents.

Due to the high tendency of alkylaldehyde to react in order to give rise to the aldol autocondensation product, an excess of benzaldehyde or benzaldehyde substituted in its aromatic ring with respect to the alkylaldehyde is added, for which reason the molar ratio of benzaldehyde/alkylaldehyde is normally between 1:1 and 10:1, preferably between 2:1 and 5:1.

The condensation reaction can also be carried out in the presence of solvents such as alcohols (methanol, ethanol, glycols), aliphatic hydrocarbons, such as pentane, hexane, cyclohexane, aromatic hydrocarbons such as toluene, xylenes, halogenated hydrocarbons, such as methylene chloride, chloroform, ethers such as diethyl ether, anisole and other tetrahydrofuran type solvents and acetonitrile although the condensation reaction is preferably carried out in the absence of solvents.

The type of aldehydes that take part in the process are benzaldehyde, or a benzaldehyde substituted in its aromatic ring in which the aromatic ring may be substituted by linear or branched alkyl or alkoxy groups, with 1 to 5 carbon atoms and that are preferably inert to the condensation reaction, such as for example methyl, ethyl, propyl, isopropyl, methoxy, ethoxy or propoxy groups. Suitable substituted benzaldehydes are for example para-isopropylbenzaldehyde and para-isobutylbenzaldehyde.

Alkylaldehydes are normally of the RCHO type, wherein R may be linear or branched, saturated or unsaturated and with a number of carbon atoms between 2 and 20. Alkylaldehydes that comprise between 2 and 8 carbon atoms, such as for example ethanal, propanal, heptanal and octanal are particularly suitable.

In an advantageous embodiment of the invention, benzaldehyde is reacted with heptanal.

The total mass of the reagents can be added from the beginning of the reaction or else discontinuously (in periodic aliquots) or continuously (with constant flow and at an addition rate of between 0.1 and 550 ml/h).

In the event of using a stirred tank type reaction system, at the end of the reaction the catalyst is filtered, washed with dichloromethane and the solvent is evaporated.

The catalysts that the present invention especially refers to, as well as the possible ways to prepare them are specified hereinafter:

### Acid catalysts:

### Amorphous aluminophosphates (AlPO)

Amorphous aluminophosphates are synthesized as described by K. Kearby (2^{nd}. Int. Cong. Catal. (Editions Technip, Paris, 1961)). These materials with a high surface (between 20-400 m²g⁻¹, preferably between 75-350 m²g^{1°}) have an Al/P ratio comprised between 0.5 and 1.5.

### Amorphous silicoaluminophosphates (SAPO):

The method to synthesize these materials is a variation of the one described by K. Kearby (identical to AlPO), wherein TEOS (Si-(Oet)₄) is added to a solution in distilled water of AlCl₃6 H₂O and H₃PO₄ (at 85% richness by weight), until the Al/Si ratio reaches the desired value. The mixture is cooled at 0°C and neutralized with NH₄OH to 10% by weight until a pH between 5.4 and 6 is reached. The gel is filtered, washed repeatedly with isopropanol and dried at 90°C. Finally, the material obtained is calcined at 650°C for 3 hours. The synthesized amorphous silicoaluminophosphates have a surface between 50-350 m²g⁻¹. The Al/P ratio varies between 0.5 and 1.5 and the Al/Si ratio is between 0.15 and 10.

### Mesoporous amorphous silica-alumina (MAS)

Amorphous silica-alumina (MAS) with a high surface, between 50-750 m²g⁻¹ is used. The Si/Al ratio varies between 0 and 100. A non-restrictive example of the preparation of silica-alumina can be found in the method described by G. Bellusi, M.G. Clerici, A. Cavati, (U.S. patent 5049 536(1992))

### Basic catalysts:

### Nitrided amorphous materials: Oxynitrided materials:

The nitriding process consists of treating the different oxides with NH₃ flows at high temperatures (between 400 and 900°C) for a period of time between 3 and 72 hours. During the process oxygen atoms are replaced by nitrogen atoms, increasing the basicity of the material. The area of the starting material remains practically constant and the content by weight of nitrogen varies between 0.1 and 20% by weight.

### EMBODIMENTS OF THE INVENTION

The invention will be additionally illustrated hereinafter on the basis of the following examples.

### Example 1: Nitriding of an AlPO for the synthesis of an oxynitrided aluminophosphate (AlPON-1):

The nitriding process is carried out by treating 2 grams of amorphous aluminophosphate (AlPO) calcined at 650°C, with an Al/P ratio of 1 and an area of 260 m²g⁻¹, with a constant dry NH₃ flow of 150 ml/min at 800°C for 16 hours. When the treatment has ended, the material was heated at 160°C with a dry N₂ flow of 150 ml/min. The AlPON-1 obtained had a N₂ content of 13%.

### Example 2: Aldol condensation between benzaldehyde and 1-Heptanal in the presence of AlPO, SAPO and MAS:

A mixture of benzaldehyde and 1-heptanal in a molar ratio of 5:1, respectively, is added to a three mouth flask provided with a cooler, under a N₂ atmosphere. The system is heated, at atmospheric pressure, at a temperature of 125°C, under magnetic stirring and 10% by weight of the total weight of AlPO (P/Al ratio =1 and area: 260 m²/g) is added. After 4 hours of reaction (the total conversion is in the range of 100%), the crude is filtered, the excess of benzaldehyde is evaporated by vacuum distillation and it is analyzed by gas chromatography-mass spectrometry (GC-MS) and ¹H-NMR. The molar composition of the crude proved to be: 85% crossed condensation product (α-amylcinnamaldehyde or Jasmine aldehyde (1)) and 15% autocondensation product of 1-heptanal (2-pentyl -2-nonenaldehyde, (2)).

Table I includes the results obtained when the condensation reaction was carried out in the presence of acid catalysts: SAPO, MAS and the corresponding oxynitrided catalysts (AlPON, SAPON and SAMON). Table 1 also includes the comparative effects, the results obtained in the absence of catalyst. In all cases a conversion of 100% was obtained.

**Table 1**

| Catalyst | N(%)^{a} | Yield 1(%) | Yield 2(%) | Others |
|---|---|---|---|---|
| AlPO | - | 85 | 15 | - |
| SAPO | - | 76 | 24 | - |
| MAS | - | 63 | 32 | 5 |
| AlPON | 13 | 77 | 20 | 3 |
| SAPON | 10 | 78 | 21 | 1 |
| SAMON | 6 | 74 | 15 | 11 |
| - | - | - | - | 4 |

| | | | | |
|---|---|---|---|---|
| ^{a} % of nitrogen by weight | | | | |

### Example 3: Influence of molar ratio benzaldehyde: 1-heptanal, using AlPO as a catalyst:

The reaction is carried out in the same conditions as in the preceding example, keeping the amount of AlPO constant (P/Al ratio = 1 and area: 260 m²/g) and varying the molar ratio benzaldehyde:1-heptanal (Benzal:Hepta) from 3:1 to 9:1. The results obtained are included in the following Table:

**Table 2**

| Molar ratio Benz:Hepta | Conv (%) | Yield 1(%) | Yield 2(%) | Others |
|---|---|---|---|---|
| 3:1 | 100 | 75 | 25 | |
| 5:1 | 100 | 85 | 15 | - |
| 7:1 | 100 | 85 | 15 | - |
| 9:1 | 95 | 81 | 13 | 1 |

### Example 4: Influence of the method of addition of 1-heptanal:

Benzaldehyde and a mass of catalyst corresponding to 8.5% by weight of AlPO (P/Al = 1, S: 260 m²/g.) are added to a three mouth flask provided with refrigerant. The system is heated under a N₂ atmosphere at a temperature of 125°C, under magnetic stirring. Then the amount of 1-heptanal corresponding to a molar ratio benzaldehyde:1-heptanal of 1.5:1 is added to the system. The addition of 1-heptanal is carried out in two different ways:
Process **A**: All the heptanal is added at the beginning of the reaction.
Process **B**: It is added in a continuous flow at an addition rate of 0.62 ml/h (addition with a perfusion pump). When the reaction reaches 100% of molar conversation, it is filtered and analyzed by gas chromatography-mass spectrometry (GC-MS) and N-NMR.

Table 3 shows the data obtained:

**Table 3**

| Process | Selectivity 1(%) | Yield 1(%) | Yield 2(%) | Others |
|---|---|---|---|---|
| A | 40 | 39 | 53 | 8 |
| B | 60 | 57 | 41 | 2 |

## Claims

1. A process for the preparation of an α-substituted cinnamaldehyde by a mixed aldol condensation reaction between an alkylaldehyde and a benzaldehyde or a benzaldehyde substituted in its aromatic ring, catalyzed by a solid catalyst, **characterized in that**
the catalyst is selected among at least a heterogeneous acid catalyst, at least a nitride of a heterogeneous acid and combinations thereof, and the condensation reaction is direct.

2. A process according to claim 1, **characterized in that** the catalyst is contained in a proportion of 0.05 to 30% by weight referred to the total weight of reagents.

3. A process according to claim 1 or 2, **characterized in that** the heterogeneous acid catalyst is selected among aluminum phosphates (AlPO), silicoaluminophosphates (SAPO) and mesoporous amorphous silica-alumina (MAS) and **in that** the nitride of heterogeneous acid catalyst is an oxynitride selected among oxynitrided aluminum phosphates (AlPON), oxynitrided silicoaluminophosphates (SAPON) and oxinitrided mesoporous amorphous silica-alumina (SAMON).

4. A process according to claim 3, **characterized in that** the aluminum phosphates (AlPO) and the oxinitrided aluminum phosphates (AlPON) have a surface between 20 and 400 m²g⁻¹, preferably between 75 and 350 m²g⁻¹ and have an Al/P ratio between 0.5 and 1.5.

5. A process according to claim 3, **characterized in that** the silicoaluminaphosphates (SAPO) and the oxynitrided silicoaluminophosphates (SAPON) have a surface between 50 and 350 m²g⁻¹, an Al/P ratio between 0.5 and 1.5, and an Al/Si ratio between 0.15 and 10.

6. A process according to claim 3, **characterized in that** the mesoporous amorphous silicoalumina (MAS) and the oxynitrided mesoporous amorphous silicoalumina (SAMON) have an area between 50 and 750 m²g⁻¹ and a Si/Al ratio between 0 and 100.

7. A process according to one of the claims 3 to 5, **characterized in that** the oxynitrides are obtained by treatment of the heterogeneous acid with a NH₃ flow and at a temperature between 400 and 900°C.

8. A process according to one of claims 3 to 6, **characterized in that** the oxynitrides have a nitrogen content by weight of 0.1 to 20% by weight.

9. A process according to claim 1, **characterized in that** the condensation reaction is carried out in an inert atmosphere, at a temperature between 25 and 300°C, preferably between 75 and 175°C, at a pressure between 1 and 10 atm, and a benzaldehyde or substituted benzaldehyde:alkylaldehyde ratio between 1:1 and 10:1, preferably between 2:1 and 5:1.

10. A process according to claim 1, **characterized in that** the condensation reaction is carried out in an inert atmosphere, at a temperature between 25 and 300°C, preferably between 75 and 175°C, at a pressure between 1 and 10 atm, and a molar ratio of benzaldehyde or substituted benzaldehyde:alkylaldehyde between 1:1 and 10:1, preferably between 2:1 and 5:1.

11. A process according to claim 1, 9 or 10, **characterized in that** the alkylaldehyde is heptanal.

12. A process according to claim 1, 9 or 10, **characterized in that** the benzaldehyde substituted in its aromatic ring is substituted by a substituent selected among linear and branched alkyl and alkoxy groups with a number of carbon atoms between 1 and 5.

13. A process according to claim 1, 9 or 10, **characterized in that** the alkylaldehyde is an aldehyde of formula RCHO, wherein R is a linear or branched, saturated or unsaturated hydrocarbonaceous chain with a number of carbon atoms between 2 and 20.

14. A process according to claim 1, 9, 10 or 12, **characterized in that** the substituted benzaldehyde is substituted in its aromatic ring by a substituent group selected among methyl, ethyl, propyl, isopropyl, methoxy, ethoxy and propoxy groups.

15. A process according to claim 1, 9, 10, 12 or 14, **characterized in that** the substituted benzaldehyde is selected between para-isopropylbenzaldehyde and para-isobutylbenzaldehyde.

16. A process according to claim 13, **characterized in that** the number of carbon atoms of the alkylaldehyde is between 2 and 8.

17. A process according to claim 13 or 16, **characterized in that** the alkylaldehyde is selected among the group constituted by ethanal, propanal, heptanal, octanal and combinations thereof.
